**Europäisches Patentamt**

**European Patent Office**

**Office européen des brevets**

(11) Veröffentlichungsnummer : **0 205 044**
**B1**

(12) **EUROPÄISCHE PATENTSCHRIFT**

(45) Veröffentlichungstag der Patentschrift :
14.02.90

(51) Int. Cl.⁵ : **G 01 B  7/22**, G 01 B  7/24

(21) Anmeldenummer : 86107290.8

(22) Anmeldetag : 28.05.86

(54) Hochtemperaturbeständige Dehnungsmesssysteme aus keramischen Materialien.

(30) Priorität : 10.06.85 DE 3520770

(43) Veröffentlichungstag der Anmeldung :
17.12.86 Patentblatt 86/51

(45) Bekanntmachung des Hinweises auf die Patenterteilung : 14.02.90 Patentblatt 90/07

(84) Benannte Vertragsstaaten :
AT BE CH DE FR GB IT LI LU NL SE

(56) Entgegenhaltungen :
EP-A- 0 088 278
EP-A- 0 137 565
AU-B-  532 592
FR-A- 2 501 362
GB-A- 1 470 591
GB-A- 2 068 562
GB-A- 2 083 012
US-A- 3 429 020
US-A- 4 197 753

(73) Patentinhaber : INTERATOM Gesellschaft mit beschränkter Haftung
Friedrich-Ebert-Strasse
D-5060 Bergisch-Gladbach 1 (DE)

(72) Erfinder : Klas, Ernst,Dipl.-Ing.
Bergaggerstrasse 33
D-5204 Lohmar (DE)
Erfinder : Stausebach, Dieter, Dipl.-Ing.
Reginharstrasse 28
D-5060 Bergisch Gladbach (DE)
Erfinder : Schmidt, Rudlf, Dipl.-Ing.
An der Foche 27
D-5064 Rösrath (DE)
Erfinder : Zentis, Alfred, Dipl.-Ing.
Heisterner Strasse 35
D-5180 Eschweiler (DE)
Erfinder : Schumacher, Günther
Maria-Juchacz-Strasse 44
D-5110 Alsdorf (DE)

(74) Vertreter : Fuchs, Franz-Josef, Dr.-Ing. et al
Postfach 22 13 17
D-8000 München 22 (DE)

## Beschreibung

Die vorliegende Erfindung betrifft hochtemperaturbeständige Dehnungsmeßsysteme aus keramischen Materialien nach dem Oberbegriff des Anspruchs 1. Auf vielen Gebieten der Technik müssen kleine und größere Längenänderungen, beispielsweise aufgrund von Materialdehnung, möglichst präzise gemessen werden. Für niedrige und mittlere Temperaturbereiche bis zu einigen 100 °C sind viele verschiedene Dehnungsmeßsysteme bekannt.

Aus der GB-PS-1 470 591 ist beispielsweise ein kapazitiver Dehnungsmeßaufnehmer bekannt. Auch induktive Wegaufnehmer und sogenannte Dehnungsmeßstreifen mit mäanderförmig gewickelten Leitern gehören zum Stand der Technik.

Schließlich gibt es auch für noch höhere Temperaturbereiche Dehnungsmeßaufnehmer, wie zum Beispiel in der EP-A2 0 088 278 beschrieben. Für Temperaturen jedoch, in denen Metalle als elektrisch leitende und gleichzeitig mechanisch belastete Teile nicht mehr verwendet werden können, gibt es bisher keine geeigneten Meßaufnehmer.

Aufgabe der vorliegenden Erfindung ist es daher, Vorrichtungen zur Dehnungsmessung anzugeben, welche sich für Temperaturbereiche eignen, in denen Metalle nicht mehr als mechanisch stabile Bauteile verwendet werden können. Insbesondere sollen Temperaturbereiche oberhalb von 900 °C bzw. sogar 1 000 °C berücksichtigt werden. Dabei sollen bekannte Meßprinzipien und Vorrichtungen durch geeignete Maßnahmen entsprechend ertüchtigt werden.

Zur Lösung dieser Aufgabe wird eine Vorrichtung zur Dehnungsmessung nach dem Anspruch 1 vorgeschlagen. Der entscheidende Schritt ist dabei der Übergang von metallischen Werkstoffen zu keramischen Materialien, welche trotzdem eine entsprechende elektrische Leitfähigkeit besitzen. In den letzten Jahren wurde beispielsweise die Entwicklungsarbeit bezüglich kohlenstoffaserverstärktem Kohlenstoff, im folgenden immer mit CFC bezeichnet, weit vorangetrieben. CFC ist inzwischen zu einem gut handhabbaren flexiblen Material von hoher Festigkeit im Bereich hoher Temperaturen geworden. Dieser Werkstoff, der zunächst für die Verwendung an großen Bauteilen, beispielsweise Rohrleitungen und Tragstrukturen, entwickelt wurde, eignet sich überraschenderweise auch für sehr kleine Bauteile und kann dort anstelle der bisher verwendeten Metalle auch im Bereich von höchsten Temperaturen eingesetzt werden. Auch andere inzwischen entwickelte leitfähige Keramiken eignen sich für solche Aufgaben. Durch Verwendung von elektrisch isolierenden keramischen Halterungen für die CFC-Bauteile lassen sich Meßgeräte nach an sich bekannten Meßprinzipien ohne die Verwendung von metallischen Werkstoffen aufbauen.

In weiterer Ausgestaltung der Erfindung wird im Anspruch 2 vorgeschlagen, daß die Halterungen in die keramische Masse eingelassene metallische Stifte aufweisen, die zur Befestigung an der Struktur bzw. als Befestigungspunkte für elektrische Zuleitungen dienen. Bei der Messung a metallischen Strukturen ist es oft günstig, die Meßaufnehmer durch solche metallischen Stifte zu befestigen, und auch die Zuleitungen benötigen Befestigungspunkte zur Entlastung und zur Verbindung mit den empfindlichen Bauteilen der Meßvorrichtung selbst.

Alternativ dazu ist eine Befestigung an der Struktur jedoch auch durch keramische Befestigungsbolzen möglich, die durch etwas konisch verengte Löcher in die Struktur eingeschossen werden. Eine entsprechende Ausführung der Halterungen wird im Anspruch 3 vorgeschlagen.

Für Einsatzfälle, in denen keine inerte Umgebungsatmosphäre vorliegt, müssen die elektrisch leitenden Bauteile natürlich aus einer oxidationsfesten Keramik bestehen, wie sie beispielsweise durch Beschichten von CFC mit Siliziumcarbid erreichbar ist. Unter Umständen genügt auch die Abdeckung mit einer dichten Kappe ohne sonstige Maßnahmen, wobei der Sauerstoff innerhalb der Kappe für eine wesentliche Schädigung des CFC nicht ausreicht. Ggf. kann das Innere der Kappe aber auch inertisiert werden.

Im Anspruch 5 wird in besonderer Ausgestaltung der Erfindung vorgeschlagen, daß die elektrisch leitenden Bauteile einen Kondensator bilden, dessen Kapazität vom Abstand der zugehörigen Halterungen abhängt. Dieses Meßprinzip und der grundsätzliche Aufbau eines solchen Meßgebers sind bekannt, jedoch müssen die Besonderheiten der neuen Werkstoffe berücksichtigt werden.

Dementsprechend wird im Anspruch 6 ein geeigneter Aufbau vorgeschlagen. Dazu werden die Kondensatorplatten direkt aus zwei nahezu parallel angeordneten, gebogenen, elektrisch leitenden Keramikplatten gebildet, welche jeweils auf beiden Seiten in paßgenauen Öffnungen in den Halterungen befestigt sind. Anders als beim Stand der Technik werden an den elastischen Bügeln keine gesonderten Kondensatorplatten mehr befestigt, sondern die Bügel selbst bilden gleichzeitig die Kondensatorplatten. Dies erfordert zwar die Aufnahme einer Eichkurve der Kapazität in Abhängigkeit vom Abstand der Halterungen, vereinfacht jedoch den Aufbau des Aufnehmers und verringert die Zahl der notwendigen Einzelteile.

Gemäß Anspruch 7 ist es auch möglich, einen Dehnungs- bzw. Wegaufnehmer mittels eines verstimmbaren Spulensystems zu bilden, wobei die Induktion der Spulen vom Abstand der zugehörigen Halterungen abhängt. Auch hier eröffnet die Verwendung von neuen Materialien höhere Temperaturbereiche. Vorteilhafte Ausgestaltungen dieser Ausführungsform sind in den Ansprüchen 9 und 10 beschrieben und werden auch anhand der Zeichnung noch näher erläutert.

Die neuen Materialien eignen sich auch zur Anwendung für das Prinzip eines Freigitter-Dehnmeßstreifens. Dabei werden die nach dem Stand der Technik verwendeten metallischen, mäanderförmig verlegten Leiter durch elektrisch leitende Fasern aus CFC oder reinem Graphit ersetzt. Die seitlichen Halterungen werden in ihrer Form entsprechend angepaßt. Gemäß Anspruch 12 besteht eine besonders günstige Ausgestaltungsform darin, daß das zur Messung dienende Freigitter aus nach dem Aufwickeln in Mäanderform verkokten Kohlefäden in geeigneten Halterungen an den Enden besteht.

Ausführungsbeispiele der Erfindung, welche nur einen kleinen Teil des weiten Anwendungsbereiches erläutern sollen, sind in der Zeichnung dargestellt. Es zeigen

Figur 1 einen erfindungsgemäßen kapazitiven Dehnungsmeßaufnehmer in Gesamtansicht,

Figur 2, 3, 4 die Einzelteile des Aufnehmers aus Figur 1,

Figur 5 die Gesamtansicht eines induktiven Wegaufnehmers,

Figur 6, 7, 8, 9, 10 Einzelteile des Wegaufnehmers in Figur 5,

Figur 11 einen vergrößerten Ausschnitt aus Figur 8,

Figur 12 die Gesamtansicht eines Freigitter-Dehnungsmeßaufnehmers und

Figur 13 einen vergrößerten Ausschnitt aus Figur 12.

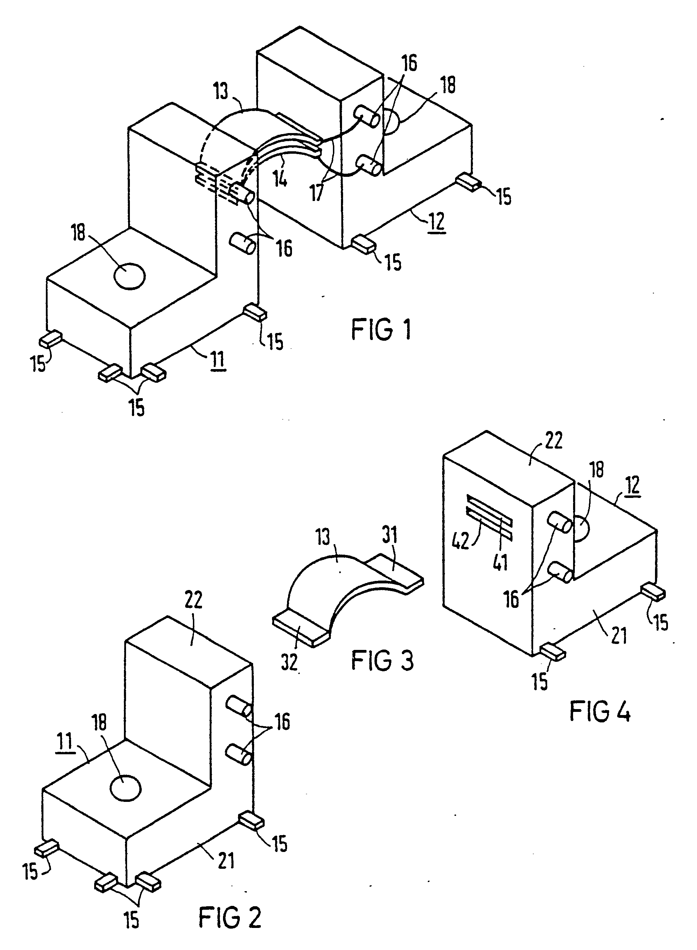

Figur 1 zeigt die Gesamtansicht eines erfindungsgemäßen kapazitiven Dehnungsmeßaufnehmers für hohe Temperaturen. Der Aufnehmer besteht aus zwei winkelförmigen Halterungen 11, 12, welche identisch oder spiegelsymmetrisch sein können, und aus zwei den Kondensator bildenden gebogenen Streifen 13, 14 aus elektrisch leitendem keramischem Material, vorzugsweise CFC. Die Einzelteile sind in den Figuren 2, 3 und 4 nochmals mit Einzelheiten dargestellt. Jede der Halterungen 11, 12 weist an der zur Auflage an einer Struktur bestimmten Seite in die Keramik eingelassene metallische Stifte 15 auf, welche zur Befestigung an der Struktur, beispielsweise durch Schweißpunkte dienen. Zusätzlich oder alternativ dazu sind in den auf die Struktur aufsetzbaren Schenkeln der Halterungen 11, 12 sich nach unten leicht konisch verengende Bohrungen 18 vorgesehen, welche eine Befestigung durch keramische Bolzen oder dergleichen zulassen. Ferner sind in die die Kondensatorplatten tragenden Schenkel der Halterungen 11, 12 weitere Stifte 16 eingelassen, welche als Befestigungspunkte der elektrischen Zuleitungen zu deren Zugentlastung dienen. Die Kondensatorplatten 13, 14 selbst sind beide identisch aufgebaut und in paßgenaue Öffnungen 41, 42 in den Halterungen 11, 12 einsetzbar. Dazu weisen die Kondensatorplatten 13, 14 jeweils an ihren beiden Enden geeignet geformte Abschnitte 31, 32 auf. Elektrisch leitende Verbindungen 17 führen von jeder Kondensatorplatte 13, 14 zu Befestigungspunkten 16. Bei der Verwendung von CFC für die Kondensatorplatten können einige der darin verwendeten Kohlefasern einfach überstehen und als Zuleitungen 17 verwendet werden.

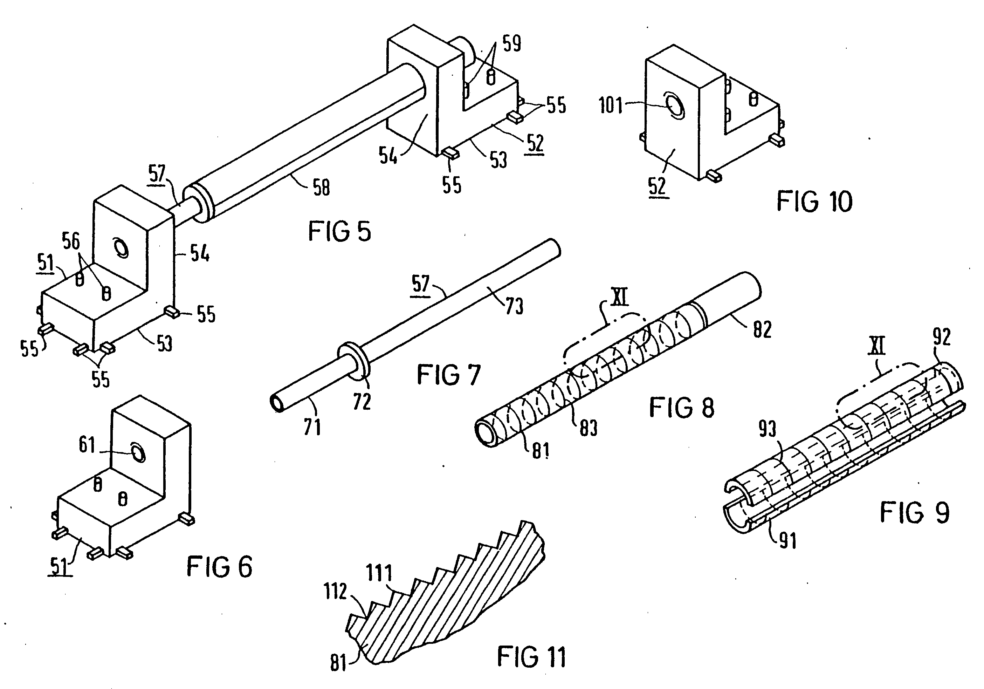

In den Figuren 5 bis 11 ist ein induktiver Wegaufnehmer mit seinen Einzelteilen dargestellt. Die obigen Ausführungen bezüglich der Befestigung von Halterungen auf einer Struktur gelten auch für die hier verwendeten winkelförmigen Halterungen 51, 52. Diese Halterungen 51, 52 können mit ihren auf der Struktur aufliegenden Schenkeln 53 mittels in das keramische Material eingebrachter metallischer Stifte 55 oder aber, wie bereits erläutert, mittels keramischer Bolzen durch geeignete, hier nicht dargestellte Löcher, befestigt werden. Auch die Funktion der Befestigungsstifte 56, 59 bleibt prinzipiell unverändert. Der induktive Wegaufnehmer besteht aus einem Spulensystem 58, welches an einer Halterung 52 befestigt ist und aus einem elektrisch leitenden Stab 57, vorzugsweise aus CFC, welcher an der anderen Halterung 51 befestigt ist und sich bei Veränderungen des Abstandes der Halterungen 51, 52 im Inneren des Spulensystems verschiebt. Durch diese Verschiebung wird die Induktion des Spulensystems verändert, was gemessen werden kann und als Maß für die Verschiebung dient. Im Ausführungsbeispiel ist der im Inneren des Spulensystems 58 verschiebbare elektrisch leitende Stab 57 aus drei Abschnitten aufgebaut. Ein Gewindeabschnitt 71 ist in ein entsprechendes Gegengewinde 61 an einem Schenkel 54 der einen Halterung 51 einschraubbar. Ein Anschlag 72 begrenzt die Eindringtiefe des zur Verstimmung des Spulensystems 58 dienenden elektrisch leitenden Abschnittes 73. Das Spulensystem selbst besteht aus einem Trägerrohr 81 und zumindest einer darauf gewickelten Spule 83. Vorzugsweise ist diese Spule in die Täler 112 einer entsprechend vorgeformten Wendel 111 des Trägerrohres 81 gewickelt. Das Trägerrohr 81 ist mittels eines Gewindeabschnittes 82 in ein entsprechendes Gegengewinde 101 in einem Schenkel einer Halterung 52 anbringbar. Da die Verstimmung nur einer Spule oft nicht mit genügender Meßgenauigkeit gemessen werden kann, wird das Spulensystem 58 vorzugsweise aus zwei Spulen aufgebaut, von denen eine als Sende- und eine als Empfangsspule dient. Diese zwei Spulen können entweder in Form einer doppelgängigen Wendel auf das Trägerrohr 81 aufgewickelt werden oder sie können, wie hier in Figur 9 angedeutet, durch eine Isolierschicht 91, 92 getrennt, konzentrisch angeordnet werden. Die Isolierschicht 91, 92 kann beispielsweise aus zwei keramischen Halbschalen bestehen, die durch die Umwicklung mit der Spule 93 zusammengehalten werden. Gegebenenfalls können auch sonstige Halterungen, beispielsweise Klammern oder dergleichen, vorgesehen werden. Die Spulen selbst können aus metallischen Leitern bestehen, da sie mechanisch nicht belastet sind. Es ist jedoch auch die Verwendung von Kohlefaser möglich.

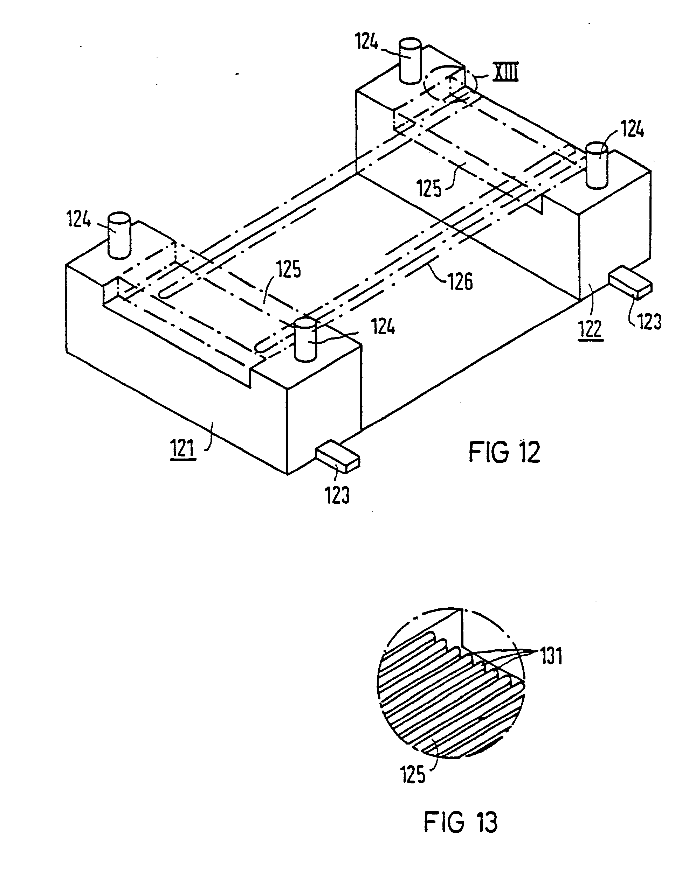

In den Figuren 12 und 13 ist ein weiteres Ausführungsbeispiel der Erfindung dargestellt. Hier wird das an sich bekannte Prinzip des Freigitter-Dehnungsmeßstreifens für die Verwendung der neuen Materialien abgewandelt. Die Halterungen 121, 122 erfüllen mit ihren eingelassenen Stiften 123, 124 die gleiche Funktion wie die entsprechenden Bauteile in den oben beschriebenen Ausführungsbeispielen. Auch die Befestigungsmöglichkeiten für die Halterungen 121, 122 sind entsprechend. Bei diesem Ausführungsbeispiel weisen die Halterungen 121, 122 auf ihrer der Struktur abgewandten Seite ein Gerüst 125 auf, um welches ein Kohlefaden 126 mäanderförmig gewickelt werden kann. Das Gerüst 125 ist vorzugsweise kammförmig ausgebildet, wobei um seine abgerundeten Zähne 131 der Kohlefaden 126 beschädigungsfrei gelegt werden kann. Die Enden des Kohlefadens sind an zweien der Befestigungsstifte 124 angebracht. Bei einer Verschiebung der Halterungen 121, 122 gegeneinander verändert sich der Widerstand bzw. die Induktivität des Kohlefadens, was gemessen werden kann. Für Anwendungsfälle, in denen das Meßsystem keinen starken äußeren Einflüssen ausgesetzt ist, ist es möglich, einen Kohlefaden mäanderförmig aufzuwickeln und anschließend zu verkoken, so daß er formstabil bleibt. In diesem Falle können die Halterungen sehr einfach ausgeführt werden und brauchen nur zur Befestigung der Kohlefadenenden und der Zuleitungen geeignet zu sein.

Die vorbeschriebenen Meßaufnehmer sind lediglich Ausführungsbeispiele für die verschiedenen Anwendungsmöglichkeiten der Erfindung. Die Verwendung anderer keramischer Materialien mit vergleichbaren oder besseren Eigenschaften, insbesondere in Bezug auf die Oxidationsfestigkeit, ist möglich und für verschiedene Anwendungsfälle nötig. Die Meßaufnehmer eignen sich für Temperaturen, in denen metallische Werkstoffe mit mechanischer Belastung nicht mehr einsetzbar sind.

**Patentansprüche**

1. Vorrichtung zur Dehnungsmessung, bestehend aus mit elektrisch leitfähigen Bauteilen (13, 14 ; 73, 83, 93 ; 126) versehenen Halterungen (11, 12 ; 51, 52 ; 121, 122), mit den Merkmalen :
a) die Halterungen (11, 12 ; 51, 52 ; 121, 122) sind auf einer Struktur an wenigstens zwei Punkten anbringbar und durch die Dehnung der Struktur gegeneinander verschiebbar ;
b) die elektrischen Eigenschaften der Anordnung der elektrisch leitfähigen Bauteile (13, 14 ; 73, 83, 93 ; 126) sind durch die Verschiebung beeinflußbar ;
gekennzeichnet durch die Merkmale :
c) die Halterungen (11, 12 ; 51, 52 ; 121, 122) bestehen zumindest teilweise aus elektrisch isolierender Keramik, vorzugsweise Aluminiumoxid ;
d) die elektrisch leitfähigen Bauteile (13, 14 ; 73, 83, 93 ; 126) bestehen mindestens zu einem Teil, der diejenige Bauteile (13, 14 ; 73, 83, 93 ; 126) umfaßt, die durch die Verschiebung mechanischen Beanspruchungen aussetzbar sind, aus elektrisch leitfähiger Keramik, vorzugsweise kohlenstoffaserverstärktem Kohlenstoff CFC.

2. Vorrichtung nach Anspruch 1, dadurch gekennzeichnet, daß die Halterungen (11, 12 ; 51, 52 ; 121, 122) in die keramische Masse eingelassene metallische Stifte (15 ; 55 ; 123 bzw. 16 ; 56, 59 ; 124) aufweisen, die zur Befestigung an der Struktur bzw. als Befestigungspunkte (16 ; 56 ; 59 ; 124) für elektrische Zuleitungen dienen.

3. Vorrichtung nach Anspruch 1 oder 2, dadurch gekennzeichnet, daß die Halterungen (11, 12 ; 51, 52 ; 121, 122) zur Struktur hinweisende, sich etwas konisch verengende Löcher (18) zur Aufnahme keramischer Befestigungsbolzen aufweisen.

4. Vorrichtung nach Anspruch 1, 2 oder 3, dadurch gekennzeichnet, daß die elektrisch leitfähigen Bauteile (13, 14 ; 73, 83, 93 ; 126) aus einer oxidationsfesten Keramik bestehen, beispielsweise mit Siliziumcarbid SiC beschichtetem CFC.

5. Vorrichtung nach Anspruch 1, 2, 3 oder 4, dadurch gekennzeichnet, daß die elektrisch leitfähigen Bauteile (13, 14) einen Kondensator bilden, dessen Kapazität vom Abstand der zugehörigen Halterungen (11, 12) abhängt.

6. Vorrichtung nach Anspruch 5, dadurch gekennzeichnet, daß die Kondensatorplatten aus zwei nahezu parallel angeordneten gebogenen elektrisch leitfähigen Keramikplatten (13, 14) bestehen, vorzugsweise aus CFC, welche jeweils auf beiden Seiten in paßgenauen Öffnungen (41, 42) in den Halterungen (11, 12) befestigt sind.

7. Vorrichtung nach Anspruch 1, 2, 3 oder 4, dadurch gekennzeichnet, daß die elektrisch leitfähigen Bauteile (73, 83, 93) ein verstimmbares Spulensystem bilden, wobei die Induktion der Spulen (83, 93) vom Abstand der zugehörigen Halterungen (51, 52) abhängt.

8. Vorrichtung nach Anspruch 7, dadurch gekennzeichnet, daß ein elektrisch leitfähiger Stab (73), vorzugsweise aus CFC, so an der einen Halterung (51) befestigt, vorzugsweise eingeschraubt ist, daß er in das Innere eines an der anderen Halterung (52) befestigten, vorzugsweise eingeschraubten Spulensystems (83, 93) ragt und abhängig vom Abstand der Halterungen (51, 52) darin verschiebbar ist.

9. Vorrichtung nach Anspruch 7 oder 8, dadurch gekennzeichnet, daß das Spulensystem aus einer Sende- (83) und einer Empfangsspule (93) besteht, deren gegenseitige Induktion durch den Stab (73) beeinflußbar ist.

10. Vorrichtung nach Anspruch 9, dadurch gekennzeichnet, daß die Sende- (83) und die Empfangsspule (93) nach Art einer doppelgängigen Schraube auf einen Kern (81) gewickelt oder durch eine Zwischenschicht (91) voneinander isoliert übereinandergewickelt sind.

11. Vorrichtung nach Anspruch 1, 2, 3 oder 4, dadurch gekennzeichnet, daß die elektrisch leitfähiger Bauteile (126) einen Freigitter-Dehnmeßstreifen bilden, dessen elektrisch leitfähige Fasern aus CFC oder reinem Graphit bestehen.

12. Vorrichtung nach Anspruch 11, dadurch gekennzeichnet, daß das Freigitter aus nach dem Aufwickeln in Mäanderform verkokten Kohlefäden besteht.

## Claims

1. Device for strain measurement, consisting of holders (11, 12 ; 51, 52 ; 121, 122), fitted with electrically conductive components (13, 14 ; 73, 83, 93 ; 126), having the characteristics :

a) the holders (11, 12 ; 51, 52 ; 121, 122) are capable of being attached to a structure at least two points and of being moved relative to each other by the extension of the structure ;

b) the electrical properties of the arrangement of electrically conductive components (13, 14 ; 73, 83, 93 ; 126) are capable of being affected by the movement ;

characterised in that :

c) the holders (11, 12 ; 51, 52 ; 121, 122) are composed, at least in part, of electrically insulating ceramic, preferably aluminium oxide ;

d) the electrically conductive components (13, 14 ; 73, 83, 93 ; 126) are composed, at least in one part which comprises those components (13, 14 ; 73, 83 ; 93 ; 126) which may be exposed to mechanical stresses as a result of the movement, of electrically conductive ceramic, preferably carbon fibre reinforced carbon, CFC.

2. Device as in Claim 1, characterised in that the holders (11, 12 ; 51, 52 ; 121, 122) have metal pins (15 ; 55 ; 123 or 16 ; 56, 59 ; 124) embedded in the ceramic substance, which are used for affixing to the structure or which serve as attachment points (16 ; 56 ; 59 ; 124) for electrical leads.

3. Device as in either of Claims 1 or 2, characterised in that the holders (11, 12 ; 51, 52 ; 121, 122) have somewhat tapering holes (18), pointing towards the structure, for accommodating ceramic fastening bolts.

4. Device as in any one of Claims 1, 2 or 3, characterised in that the electrically conductive components (13, 14 ; 73, 83, 93 ; 126) are composed of a non-oxidizing ceramic, for example silicon carbide SiC coated CFC.

5. Device as in any one of Claims 1, 2, 3 or 4, characterised in that the electrically conductive components (13, 14) form a capacitor, the capacitance of which depends on the separation of the associated holders (11, 12).

6. Device as in Claim 5, characterised in that the capacitor plates consist of two curved electrically conductive ceramic plates (13, 14), preferably composed of CFC, in virtually parallel arrangement, each of which is secured at both ends in precision-fit apertures (41, 42) in the holders (11, 12).

7. Device as in any one of Claims 1, 2, 3 or 4, characterised in that the electrically conductive components (73, 83, 93) form a detunable coil system in which the induction of the coils (83, 93) depends on the separation of the associated holders (51, 52).

8. Device as in Claim 7, characterised in that an electrically conductive rod (73), preferably composed of CFC, is secured to one holder (51), preferably screwed in, in such a manner that it projects into the interior of a coil system (83, 93) secured to the other holder (52), preferably screwed in, and is capable of movement within the coil system, depending on the separation of the holders (51, 52).

9. Device as in either of Claims 7 or 8, characterised in that the coil system consists of a transmitting coil (83) and a receiving coil (93), the mutual induction of which is influenceable by the rod (73).

10. Device as in Claim 9, characterised in that the transmitting coil (83) and the receiving coil (93) are wound, in the manner of a double-threaded screw, on a core (81) or are wound, superimposed on one another, isolated one from the other by means of an intermediate layer (91).

11. Device as in any one of Claims 1, 2, 3 or 4, characterised in that the electrically conductive components (126) form a floating grid strain gauge, the electrically conductive fibres of which are composed of CFC or pure graphite.

12. Device as in Claim 11, characterised in that the floating grid consists of carbon threads coked after winding in meander form.

## Revendications

1. Dispositif extensométrique, constitué par des supports (11, 12 ; 51, 52 ; 121, 122) comportant des composants électriquement conducteurs (13, 14 ; 73, 83, 93 ; 126), présentant les caractéristiques suivantes :

a) les supports (11, 12 ; 51, 52 ; 121, 122) peuvent être montés en au moins deux points sur une structure et sont déplaçables l'un par rapport à l'autre sous l'effet de l'allongement de la structure ;

b) les caractéristiques électriques de l'ensemble formé par les composants électriquement conducteurs (13, 14 ; 73, 83, 93, 126) peuvent être influencés par le déplacement ;

remarquable par les caractéristiques suivantes :

c) les supports (11, 12 ; 51, 52 ; 121, 122) sont constitués au moins en partie par une céramique électriquement isolante, de préférence de l'oxyde d'aluminium ;

d) des composants électriquement conducteurs (13, 14 ; 73, 83, 93 ; 126) sont constitués, au moins pour une partie entourant les composants (13, 14 ; 73, 83, 93 ; 126) qui peuvent être soumis à des contraintes mécaniques sous l'effet du déplacement, par une céramique électriquement conductrice, de préférence du carbone renforcé par des fibres de carbone CFC.

2. Dispositif suivant la revendication 1, caractérisé par le fait que les supports (11, 12 ; 51, 52 ; 121, 122) comportent des tiges métalliques (15 ; 55 ; 123 ou 16 ; 56, 59 ; 124), qui sont utilisées pour la fixation sur la structure ou comme points de fixation (16 ; 56 ; 59 ; 124) pour des conducteurs d'alimentation électrique.

3. Dispositif suivant la revendication 1 ou 2, caractérisé par le fait que les supports (11, 12 ; 51, 52 ; 121, 122) comportent des trous (18), qui sont dirigés vers la structure, se rétrécissent avec une forme légèrement conique et servent à loger des boulons de fixation céramiques.

4. Dispositif suivant la revendication 1, 2 ou 3, caractérisé par le fait que les composants électriquement conducteurs (13, 14 ; 73, 83, 93 ; 126) sont constitués par une céramique résistante à l'oxydation, par exemple du CFC recouvert de carbure de silicium SiC.

5. Dispositif suivant la revendication 1, 2, 3 ou 4, caractérisé par le fait que les composants électriquement conducteurs (13, 14) forment un condensateur, dont la capacité dépend de la distance des supports associés (11, 12).

6. Dispositif suivant la revendication 5, caractérisé par le fait que les plaques du condensateur sont constituées par deux plaques céramiques électriquement conductrices coudées (13, 14), réalisées de préférence en CFC, qui sont approximativement parallèles et sont fixées respectivement des deux côtés dans des ouvertures (41, 42) adaptées de façon précise et ménagées dans les supports (11, 12).

7. Dispositif suivant la revendication 1, 2, 3 ou 4, caractérisé par le fait que les composants électriquement conducteurs (73, 83, 93) forment un système de bobines pouvant être désaccordé, l'induction des bobines (83, 93) dépendant de la distance des supports associés (51, 52).

8. Dispositif suivant la revendication 7, caractérisé par le fait qu'une barre électriquement conductrice (73), réalisée de préférence en CFC, est fixée sur l'un (51) des supports, de préférence par vissage, de sorte qu'elle pénètre à l'intérieur d'un système de bobine (83, 93) fixé sur l'autre support (52), de préférence par vissage, et peut être déplacée dans ce système en fonction de la distance des supports (51, 52).

9. Dispositif suivant la revendication 7 ou 8, caractérisé par le fait que le système de bobines est constitué par une bobine d'émission (83) et une bobine de réception (93), dont l'induction mutuelle peut être influencée au moyen de la barre (73).

10. Dispositif suivant la revendication 9, caractérisé par le fait que la bobine d'émission (83) et la bobine de réception (93) sont enroulées à la manière d'une hélice à deux filets sur un noyau (81) ou sont enroulées en superposition, en étant isolées l'une par rapport à l'autre par une couche intercalaire (91).

11. Dispositif suivant les revendications 1, 2, 3 ou 4, caractérisé par le fait que les composants électriquement conducteurs (126) forment une jauge extensométrique à grille libre, dont les fibres électriquement conductrices sont constituées par du CFC ou du graphique pur.

12. Dispositif suivant la revendication 11, caractérisé par le fait que la grille libre est constituée par des fils de carbone carbonisés après enroulement sous une forme sinueuse.

FIG 1

FIG 2

FIG 3

FIG 4

FIG 10

FIG 9

FIG 8

FIG 5

FIG 7

FIG 6

FIG 11

2

FIG 12

FIG 13

3